## Europäisches Patentamt

## European Patent Office

## Office européen des brevets

(11) Veröffentlichungsnummer: **0 212 127**
**B1**

(12) **EUROPÄISCHE PATENTSCHRIFT**

(45) Veröffentlichungstag der Patentschrift:
**30.08.89**

(51) Int. Cl.⁴: **A 61 M 1/34**

(21) Anmeldenummer: **86108318.6**

(22) Anmeldetag: **19.06.86**

(54) Vorrichtung zur Hämodiafiltration.

(30) Priorität: **22.08.85 DE 3529973**

(43) Veröffentlichungstag der Anmeldung:
**04.03.87 Patentblatt 87/10**

(45) Bekanntmachung des Hinweises auf die Patenterteilung: **30.08.89 Patentblatt 89/35**

(84) Benannte Vertragsstaaten:
**AT BE CH DE FR GB IT LI LU NL SE**

(56) Entgegenhaltungen:
**EP–A– 0 122 604**
**DE–A– 2 755 882**
**DE–A– 3 111 061**
**FR–A– 2 332 031**
**FR–A– 2 457 694**

(73) Patentinhaber: **B. Braun-SSC AG**
**Gerilswilstrasse 74**
**CH-6020 Emmenbrücke (CH)**

(72) Erfinder: **Heitmeier, Rolf**
**Göthestrasse 8**
**D-3507 Baunatal (DE)**
Erfinder: **Rath, Dieter**
**Franz-Gleim-Strasse 69**
**D-3508 Melsungen (DE)**

(74) Vertreter: **von Kreisler, Alek, Dipl.-Chem. et al**
**Deichmannhaus am Hauptbahnhof**
**D-5000 Köln 1 (DE)**

## Beschreibung

Die Erfindung betrifft eine Vorrichtung zur Hämodiafiltration mit einem Hämodiafilter, dessen erste Volumenkammer mit dem Blutkreislauf eines Patienten verbindbar ist und dessen zweite Volumenkammer an einem externen Flüssigkeitsweg angeschlossen ist.

Während bei der Hämodialyse das Blut urämischer Patienten über eine semipermeable Membran entgiftet wird, auf deren beiden Seiten eine Konzentrationsdifferenz der gelösten Substanzen besteht, wird bei der Hämodiafiltration ein Hämodiafilter benutzt, das größere Moleküle durchläßt und einen hohen konvektiven Stofftransport durch die Membran ermöglicht. Im Unterschied zur Dialyse, bei der durch die Konzentrationsdifferenz zwischen Dialyseflüssigkeit und Blut dem Blut nur bestimmte Bestandteile entzogen werden, passieren bei der Hämodiafiltration auch solche Blutbestandteile die Filtermembran, bei denen keine Konzentrationsdifferenz zwischen Einlaßseite und Auslaßseite des Filters vorhanden ist. Bei der Hämodiafiltration werden daher dem Blut erheblich mehr Bestandteile entzogen als bei der Dialyse. Um bei der notwendigerweise vorhandenen hohen Ultrafiltrationsrate eine Eindickung des Blutes zu verhindern, ist die Zudosierung einer Substitutionslösung zum Patienten erforderlich.

Bei einer bekannten Vorrichtung zur Hämodiafiltration (DE-A-32 13 390) wird zur Bestimmung der Reinfusionsmenge eine Waage benutzt, mit der die Masse des Filtrats bestimmt wird, und in Abhängigkeit von der Filtratmasse wird ebenfalls durch Wägung die dem Patienten zuzuführende Substitutionslösungsmenge dosiert.

Bei einer bekannten Dialyseeinrichtung (DE-A-32 02 831) ist in dem Flüssigkeitsweg vor dem Dialysefilter eine volumengeregelte Pumpe und hinter dem Dialysefilter eine in Abhängigkeit vom Transmembrandruck geregelte Saugpumpe vorhanden. Die Saugpumpe hält den Transmembrandruck konstant. Diese Dialysevorrichtung eignet sich nicht für die Hämodiafiltration, weil einerseits die bei der Dialyse eingesetzte Filtermembran für größere Moleküle undurchlässig ist und andererseits keine Möglichkeit besteht, die Eindickung des Blutes des Patienten zu verhindern.

Der Erfindung liegt die Aufgabe zugrunde, eine Vorrichtung der eingangs genannten Art zu schaffen, die ohne Benutzung eines Wägesystems oder eines Flußmeßsystems eine Hämodiafiltration ermöglicht und bei der dem Patienten kontinuierlich die erforderliche Substitutionslösungsmenge zugeführt wird.

Die Lösung dieser Aufgabe besteht erfindungsgemäß darin, daß der externe Flüssigkeitsweg vor dem Filter eine erste volumengeregelte Pumpe und hinter dem Filter eine in Abhängigkeit von Transmembrandruck geregelte Saugpumpe aufweist und daß in den Flüssigkeitsweg vor dem Filter ein Abzweig vorgesehen ist, der über eine zweite Pumpe mit einem den Patienten und die Eingangsseite des Filters enthaltenden Kreislauf verbunden ist.

Die Saugpumpe ist, ähnlich wie bei dem erwähnten bekannten Dialysator, in Abhängigkeit vom Transmembrandruck geregelt, um diesen Transmembrandruck konstant zu halten. Bei konstantem Transmembrandruck wird das Filter von einer konstanten Filtratmenge passiert, so daß dem patientenseitigen Kreislauf pro Zeiteinheit eine konstante Flüssigkeitsmenge entzogen wird. Diese Flüssigkeitsmenge wird dem Kreislauf durch die zweite Pumpe, anhand eines speziellen Abgleichvorganges, kontinuierlich zugeführt. Als Substitutionslösung wird hierbei die Flüssigkeit des externen Flüssigkeitsweges benutzt. Auf diese Weise wird durch Mengenregelung unter Vermeidung von Wägesystemen die Blutkonzentration des Patienten konstant gehalten. Fernerhin ist es vorteilhaft, daß in dem externen Flüssigkeitsweg eine Meßkammer gemäß der Beschreibung DE-A-32 02 831 existiert.

Die erfindungsgemäße Vorrichtung zur Hämodiafiltration kann auch für die Hämodialyse benutzt werden, wobei dann allerdings die Membran des Filters durch eine für die Hämodialyse geeignete Membran ersetzt werden muß. Außerdem ist der Abzweig zu sperren bzw. die zweite Pumpe abzuschalten.

Gemäß einer bevorzugten Ausführungsform der Erfindung ist eine dritte Pumpe in dem Kreislauf zwischen dem Patienten und vor dem Filter vorgesehen. Die dritte Pumpe dient zur Aufrechterhaltung des Blutstromes im Patientenkreislauf.

Da sich während einer Hämodiafiltration die Druck- und Mengenverhältnisse dadurch ändern können, daß die Filtermembran sich zusetzt, ist es zweckmäßig, in bestimmten zeitlichen Abständen jeweils einen Abgleichzyklus durchzuführen, um die Ultrafiltrationsrate zu kontrollieren und den Transmembrandruck durch Veränderung der Saugkraft der Saugpumpe zu korrigieren. Hierzu ist der Flüssigkeitsweg an einen Meßbehälter angeschlossen und es ist eine Kurzschlußleitung vorgesehen, um den Flüssigkeitsweg vorübergehend zu einem Kreislauf kurzzuschließen. Die Zeitdauer zum Leeren bzw. Füllen des Meßbehälters dient zur Bestimmung des neu einzustellenden Transmembrandrucks.

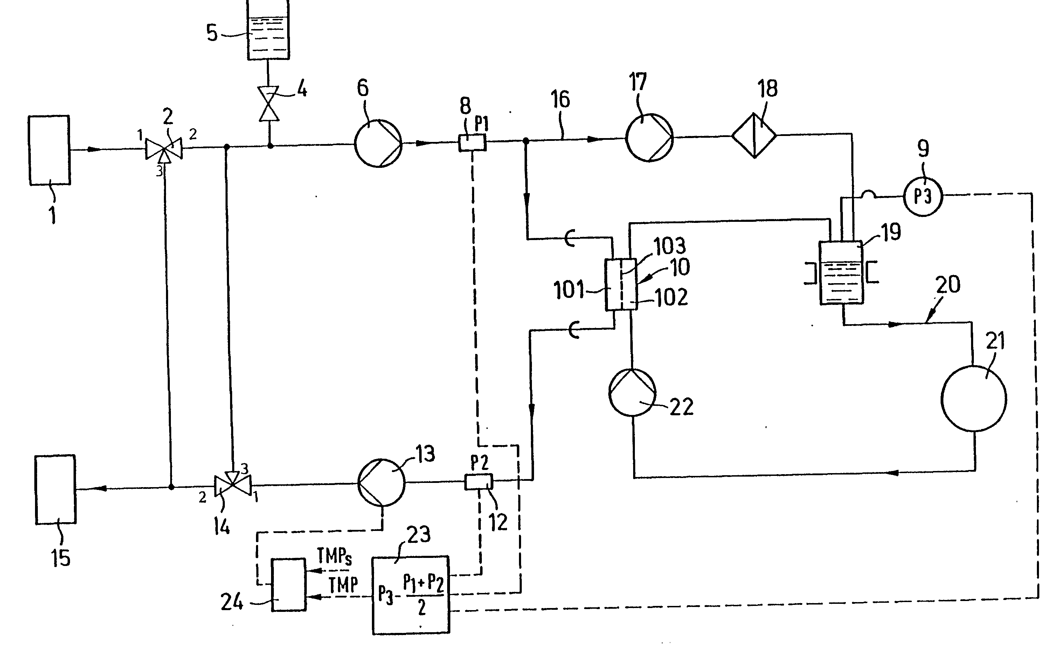

Im folgenden wird unter Bezugnahme auf die einzige Figur der Zeichnung ein Ausführungsbeispiel der Erfindung näher erläutert.

In der Zeichnung ist ein Prinzip-Schaltbild der Vorrichtung zur Hämodiafiltration dargestellt.

An eine Flüssigkeitsquelle 1 ist über ein Umschaltventil 2 die volumengeregelte Pumpe 6 angeschlossen. Diese Förderpumpe ist über ein Druckmeßorgan 8, das den Druck $P_1$ mißt, mit dem einen Ende der auslaßseitigen Kammer 101 des Filters 10 verbunden. Das andere Ende dieser Kammer 101 ist über einen weiteren Druckfühler 12, der den Druck $P_2$ mißt, mit der Saugseite der Saugpumpe 13 verbunden. Die Auslaßseite der Saugpumpe 13 ist über das Umschaltventil 14 an den Ablauf 15 angeschlossen.

Von dem Weg von der volumengeregelten Pumpe 6 zur Kammer 101 des Filters 10 führt ein Abzweig 16 zu einer zweiten Pumpe 17, die über ein Sterilfilter 18 mit dem Pufferbehälter 19 verbunden ist. Der Pufferbehälter 19 ist Bestandteil des patientenseitigen Kreislaufs 20, der außerdem das Blutsystem des Patienten 21, eine dritte Pumpe 22 und die einlaßseitige Kammer 102 des Filters 10 enthält. Der Kreislauf 20 ist ein geschlossener Kreislauf, d. h. der Pufferbehälter 19 ist gegenüber der Außenluft vollständig abgedichtet. Er hat eine transparente Wand und dient gleichzeitig als Luftdetektor. Wenn in dem Kreislauf 20 Luft vorhanden ist, fällt der Flüssigkeitsstand im Pufferbehälter 19. Durch Überwachung des Flüssigkeitsstandes kann die Luftmenge überwacht werden. Der Pufferbehälter 19 ist außerdem an einen Druckmeßfühler 9 angeschlossen, der den Druck $P_3$ mißt.

Aus den Drücken $P_1$, $P_2$ und $P_3$ wird in der Rechenschaltung 23 der Transmembrandruck TMP an der Filtermembran nach der Beziehung

$$TMP = P_3 - (P_1 + P_2)/2$$

ermittelt. In einer Vergleichsschaltung 24 wird der Wert TMP mit dem voreingestellten Sollwert $TMP_s$ für den mittleren Transmembrandruck verglichen und die Differenz der beiden Werte wird zur Regelung der Saugpumpe 13 benutzt. Auf diese Weise wird erreicht, daß die Saugpumpe 13 stets eine solche Saugkraft entwickelt, daß an dem Filter 10 der Transmembrandruck TMP auf dem Sollwert $TMP_s$ gehalten wird.

Bei Start der Hämodiafiltration werden die Ventile 2 und 14 in Richtung 1/3 durchströmt. Fernerhin werden in dieser Situation die Pumpen 6 und 13 stillgesetzt, so daß ein in sich geschlossener Kreislauf auf der externen Flüssigkeitsseite des Filters entsteht.

Der geschlossene Flüssigkeitskreislauf ist die Voraussetzung, daß der für den angestrebten hohen konvektiven Stofftransport notwendige Transmembrandruck ermittelt werden kann. Fernerhin wird hierdurch die Möglichkeit geschaffen, die Durchlässigkeit des Filters zu bestimmen und als Filterkenngröße für die weitere Vorgehensweise zu definieren.

Dies geschieht erfindungsgemäß in der Weise, daß die zweite Pumpe 17 annähernd mit der für den angestrebten konvektiven Stofftransport notwendigen Förderrate betrieben wird. Der sich einstellende Transmembrandruck wird über die Sensoren $P_1$, $P_2$ und $P_3$ erfaßt, in der Einheit 25 als Basisdruck abgespeichert und über einen geeigneten Algorithmus wird die Filterkenngröße bestimmt.

Um neben dem reinen konvektiven Stofftransport auch die Entgiftung über das Konzentrationsgefälle zwischen Blut- und Flüssigkeitsseite zu ermöglichen, ist es notwendig, daß die Ventile 2 und 14 in Richtung 1/2 durchströmt werden und die volumengeregelte Pumpe 6 den Dialysatfluß aufbaut. In dieser Situation wird die Saugpumpe 13 durch die Einheit 24 geregelt, wobei der notwendige Transmembrandruck als $TMP_s$ von der Recheneinheit 25 unter Berücksichtigung der Filterkenngröße und dem Basisdruck vorgegeben wird. Durch diese Vorgehensweise ist der Fluß durch die Filtermembran die Summe aus Substitutionslösungsfluß plus Ultrafiltrationsfluß. Die dem Patienten über die Pumpe 17 zugeführte Substitutionslösungsmenge entspricht somit im wesentlichen der durch die Filtermembran 103 abgezogenen Flüssigkeitsmenge.

Um die Aufrechterhaltung dieses Gleichgewichts auch bei verändertem Durchlaßverhalten der Filtermembran 103 sicherzustellen, ist es erforderlich, von Zeit zu Zeit einen Abgleichzyklus durchzuführen. Hierzu werden die Ventile 2 und 14 in die Stellung 1/3 geschaltet, wodurch die Dialysatquelle 1 mit dem Ablauf 15 verbunden und außerdem der die Pumpen 6 und 13 und die Kammer enthaltenden Flüssigkeitsweg zu einem Kreislauf geschaltet wird. Das vor der Pumpe 6 abzweigende Ventil 4 wird geöffnet, so daß der Meßbehälter 5 nun an den Kreislauf angeschlossen ist. Da dem Kreislauf durch die Pumpe 17 die Substitutionslösungsmenge entzogen wird und da andererseits dem Kreislauf durch die Filtermembran 103 hindurch eine der Substitutionslösungsmenge entsprechende Flüssigkeitsmenge und zusätzlich noch eine Ultrafiltratmenge zugeführt wird, steigt der Flüssigkeitsstand in dem Meßbehälter 5. Die Schnelligkeit des Anstiegs gibt Aufschluß darüber, in welcher Weise die Steuerung der Saugpumpe 13 verändert werden muß bzw. welcher Transmembrandruck $TMP_s$ eingestellt werden muß, um eine bestimmte Ultrafiltrationsrate zu erhalten.

## Patentansprüche

1. Vorrichtung zur Hämodiafiltration mit einem Hämodiafilter (10), dessen erste Volumenkammer (102) mit dem Blutkreislauf eines Patienten verbindbar ist und dessen zweite Volumenkammer (101) an einen externen Flüssigkeitsweg angeschlossen ist, dadurch gekennzeichnet, daß der externe Flüssigkeitsweg vor dem Filter (10) eine erste volumengeregelte Pumpe (6) und hinter dem Filter (10) eine in Abhängigkeit vom Transmembrandruck geregelte Saugpumpe (13) aufweist und daß in dem Flüssigkeitsweg vor dem Filter (10) ein Abzweig (16) vorgesehen ist, der über eine zweite volumengeregelte Pumpe (17) mit einem zwischen dem Patienten und dem Filter (10) vorhandenen Kreislauf (20) verbunden ist.

2. Vorrichtung nach Anspruch 1, dadurch gekennzeichnet, daß hinter der zweiten volumengeregelten Pumpe (17) mindestens ein Sterilfilter (18) angeordnet ist.

3. Vorrichtung nach Ansprüche 1 oder 2, dadurch gekennzeichnet, daß der Kreislauf (20) einen Pufferbehälter (19) enthält, an den die zweite volumengeregelte Pumpe (17) angeschlossen ist und der einen Luftdetektor aufweist.

4. Vorrichtung nach einem der Ansprüche 1 bis 3, dadurch gekennzeichnet, daß der Flüssigkeitsweg an einen Meßbehälter (5) angeschlossen ist und daß eine Kurzschlußleitung vorgesehen ist, um den Flüssigkeitsweg vorübergehend zu einem Kreislauf kurzzuschließen.

## Claims

1. Apparatus for hemodiafiltration comprising a hemodiafilter (10) whose first volume chamber (102) is connectible to the blood circulation system of a patient and whose second volume chamber (101) is connected to an external fluid path, characterized in that the external fluid path comprises upstream of the filter (10) a first volume-controlled pump (6) and downstream of the filter (10) a suction pump (13) controlled in dependence of the transmembrane pressure and that in said fluid path, upstream of the filter (10), a branch (16) is provided which is connected via a second volume-controlled pump (17) to a circulatory system (20) provided between the patient and the filter (10).

2. Apparatus according to claim 1, characterized in that downstream of the second volume-controlled pump (17), there is provided at least one sterile filter (18).

3. Apparatus according to claim 1 or 2, characterized in that the circulatory system (20) includes a buffer container (19) to which the second volume-controlled pump (17) is connected and which is provided with an air detector.

4. Apparatus according to any one of claims 1 to 3, characterized in that the fluid path is connected to a measuring container (5) and that a short-circuit conduit is provided to temporarily short-circuit the fluid path to a circulatory system.

## Revendications

1. Dispositif d'hémodialyse comportant un filtre d'hémodialyse (10), dont la première chambre (102) peut être reliée à la circulation sanguine d'un patient et dont la seconde chambre (101) est raccordée à une voie extérieure de circulation d'un liquide, caractérisé en ce que la voie extérieure de circulation du liquide comporte une première pompe (6) à cylindrée réglée, placée en amont du filtre (10), et une pompe aspirante (13) réglée en fonction de la pression trans-membrane et placée en aval du filtre (10), et en ce qu'il est prévu dans la voie de circulation du liquide, en amont du filtre (10), une dérivation (16), qui est reliée par l'intermédiaire d'une seconde pompe à cylindrée réglée (17) à un circuit (20) présent entre le patient et le filtre (10).

2. Dispositif selon la revendication 1, caractérisé en ce qu'au moins un filtre stérile (18) est disposé en aval de la seconde pompe (17) à cylindrée réglée.

3. Dispositif selon la revendication 1 ou 2, caractérisé en ce que le circuit (20) contient un réservoir tampon (19), auquel est raccordée la seconde pompe à cylindrée réglée (17) et qui comporte un détecteur d'air.

4. Dispositif selon l'une des revendications 1 à 3, caractérisé en ce que la voie de circulation du liquide est raccordée à un récipient de mesure (5) et en ce qu'il est prévu une canalisation de court-circuit servant à court-circuiter transitoirement la voie de circulation du liquide pour former un circuit fermé.

EP 0 212 127 B1